# EUROPEAN PATENT SPECIFICATION

(11) **EP 3 222 276 B1**
(45) Date of publication and mention of the grant of the patent: **07.04.2021**
(21) Application number: 14906443.8
(22) Date of filing: 19.11.2014
(51) Int. Cl.: A61K 31/404, A61K 31/44, C12Q 1/37, A61P 35/00

(54) **PHARMACEUTICAL COMPOSITION FOR TREATING CANCER AND BIOMARKER FOR DRUG SCREENING**
PHARMAZEUTISCHE ZUSAMMENSETZUNG ZUR KREBSBEHANDLUNG UND BIOMARKER FÜR WIRKSTOFFSCREENING
COMPOSITION PHARMACEUTIQUE POUR LE TRAITEMENT DU CANCER ET BIOMARQUEUR POUR LE CRIBLAGE DE MÉDICAMENT

(43) Date of publication of application: 27.09.2017
(73) Proprietor: National Defense Medical Center, Taipei City 114 (CN)
(72) Inventor: CHA, Tai-lung, Taipei (TW); CHANG, Sun-yran, Taipei 114 (TW); SUN, Guang-huan, Taipei (TW); LIN, Chung-chih, Taipei 114 (TW); TSAI, Yi-ta, Taipei (TW)
(74) Representative: Lang, Christian
(86) International application number: PCT/CN2014/091712
(87) International publication number: WO 2016/078044

(56) References cited:
- WO-A2-2011/097594
- WO-A2-2012/021778
- US-A1- 2014 357 673
- Y.-T. TSAI ET AL: "Novel Cancer Therapeutics with Allosteric Modulation of the Mitochondrial C-Raf-DAPK Complex by Raf Inhibitor Combination Therapy", CANCER RESEARCH, vol. 75, no. 17, 1 September 2015 (2015-09-01), pages 3568-3582, XP55472032, US ISSN: 0008-5472, DOI: 10.1158/0008-5472.CAN-14-3264
- XIANG, SHALI ET AL.: 'The Tumor Suppressor Gene DAPK: a Novel Drug Treatment Target.' PROGRESS IN MODERN BIOMEDICINE. vol. 12, no. 1, 31 January 2012, pages 163 - 165, XP008185254
- SARAH BURGESS ET AL.: 'Raf Inhibitors as Therapeutic Agents Against Neurodegenerative Diseases.' CNS & NEUROLOGICAL DISORDERS-DRUG TARGETS. vol. 9, no. 1, 31 December 2010, pages 120 - 127, XP008185253

## Description

### BACKGROUND OF THE INVENTION

### 1. FIELD OF THE INVENTION

The present invention relates to a pharmaceutical composition for use in treating cancer, comprising sorafenib and GW5074. The invention also discloses a compound for dissociating a protein complex consisting of c-Raf and DAPK. Additionally, the invention further provides a method for screening a candidate drug using a biomarker.

### 2. DESCRIPTION OF THE PRIOR ART

Activation of the proto-oncogenes or deficiency of the tumor suppressor genes often leads to cancer cell development. Ras is a proto-oncogene and the activation of Ras protein is normally triggered by receptor tyrosine kinases (TKIs) on the cell membrane. Activated Ras protein binds with RAF and subsequently transmits the signal downstream to activate the MAPK pathway that, in turn, regulates the cell growth and cell differentiation. The activated Grb-sos protein arising from the binding of a growth factor to its receptor on the cell membrane leads to phosphorylation of the downstream Ras-GDP protein, and the resultant Ras-GTP then binds to the N-terminus of Raf protein and activates Rat which further regulates the activation of ERK through the phosphorylation of MEK. Next, the activated ERK enters the cell nucleus and induces cancer cell proliferation. Therefore, countless drugs that are designed specifically against these proto-oncogenes such as these tyrosine kinase inhibitors (TKIs) have been generated, only to discover that many cancer patients developed drug resistance during the treatment. Consequently, a combination therapy that specifically targets the signaling transduction pathway of tyrosine kinase has become a common treatment method.

Angiogenesis and cell proliferation play essential roles in the tumor growth. In tumor, when vast amounts of vascular endothelial growth factor A (VEGF-A) are released from cancer cells, the binding of VEGF-A with the vascular endothelial growth factor receptor 2 (VEGFR-2) on the surface of the endothelial cells of a tumor activates the signaling transduction pathway of Raf/mitogen-activated protein kinase (MEK) /extracellular signal-regulated kinase (ERK), which in turn induces the angiogenesis of endothelial cells. Meanwhile, the Ras-ERK pathway also enhances the cancer cell proliferation. In addition, the loss of regulation of the Ras-Raf-ERK pathway has been shown in a number of tumor cell lines. Thus, VEGF and Raf may be the best targets for the inhibition of tumor growth.

Sorafenib (Nexavar*, BAY 43-9006, Bayer HealthCare Pharmaceuticals) is an oral Multi-Kinase Inhibitor commonly used for treating various cancers. Sorafenib can inhibit proteins such as Raf, VEGF receptor, platelet-derived growth factor (PDGF) receptor, KIT and Fms-like tyrosine kinase-3 (FLT-3). A number of studies have Indicated that sorafenib inhibits the tumor growth by inhibiting Raf signaling pathway in different cancer cells while suppressing the proliferation of endothelia cells surrounding cancer cells through the inhibition of VEGF as well as PDGF signaling pathways, and subsequently induces cancer cell death. The design and test results of these drugs are ideal. However, after years of clinical applications, it was unfortunately discovered that, although the tumor size at the early stage of treatment was efficiently reduced by sorafenib, such drugs not only fails to eradicate the tumor completely but may also cause serious side effects. Moreover, the treated cancer cells developed drug resistance after the long-term treatment. Additionally, in recent years, certain studies have shown that the inhibition of Raf signaling pathway in cancer cells leads to reduced inhibition of sorafenib due to alternative regulations by different molecules in cancer cells. Most importantly, some evidence further indicated that the inhibition effects of sorafenib on cancer cells may not be regulated by the Raf pathway. Hence, the aforementioned deficiencies need to be further improved.

### SUMMARY OF THE INVENTION

Although sorafenib may effectively reduce the tumor size at an early stage, it cannot eradicate the tumor; further, it often comes with severe side effects, and as the treatment period increases, cancer cells often become resistant. In view of the foregoing, sorafenib is not an ideal compound for use in tumor treatment and there exists a need in the related art to provide an improved compound for use in treating cancer. In view of the foregoing, the present invention is directed to the following inventions: (1) a novel pharmaceutical composition for use in treating cancer, comprising sorafenib and GW5074; (2) a compound for use in treating cancer which causes the dissociation of c-Raf and DAPK, which leads to cell necrosis thereby effectively killing cells; and (3) a method for screening a candidate drug using a DAPK protein having the sequence of SEQ ID NO: 2 as biomarker, wherein the serine 308 (Ser308) amino acid residue of the DAPK protein is phosphorylated.

In one aspect, the present invention provides a pharmaceutical composition for use in treating cancer, comprising sorafenib or a pharmaceutically acceptable salt thereof and 3-(3,5-Dibromo-4-hydroxy-benzylidene)- 5-iodo-1,3-dihydro-indol-2-one (GW5074) or a pharmaceutically acceptable salt thereof.

According to embodiments of the present invention, the compounds of the composition are administered separately, concurrently, or sequentially.

According to embodiments of the present invention, the cancer is renal cell carcinoma, prostate cancer, breast cancer, lung cancer, cervical carcinoma, oral cancer, glioma, urothelial cell carcinoma, or melanoma.

According to embodiments of the present invention, the pharmaceutical composition further comprises a pharmaceutically acceptable vehicle for said compound(s). The aforementioned vehicles can be excipients, diluents, thickeners, fillers, binders, disintegrants, lubricants, oil or non-oil agents, surfactants, suspending agents, gelling agents, adjuvants, preservatives, antioxidants, stabilizers and coloring agents.

According to embodiments of the present invention, the pharmaceutical composition is administered via oral administration, immersion, injection, topical application or patch administration.

According to the pharmaceutical composition for use in treating cancer of the present invention, GW5074 alters the conformation of a c-Raf protein having the sequence of SEQ ID NO: 1 upon binding thereto, thereby increasing the binding affinity of the sorafenib toward the c-Raf protein so as to dissociate c-Raf from the c-Raf/DAPK protein complex.

The present invention also provides a compound comprising sorafenib and 3-(3,5-Dibromo-4-hydroxy-benzylidene)-5-iodo-1,3-dihydro-indol-2-one (GW5074) for dissociating a protein complex, wherein said protein complex consists of c-Raf (SEQ ID NO: 1) and DAPK (SEQ ID NO: 2).

In another aspect, the invention discloses a method for for screening a candidate drug using a biomarker; the method comprising, providing a specimen; detecting a phosphorylation level of the biomarker in the specimen prior to the administration of the drug, and detecting a cell growth inhibition rate of the test drug on the specimen after the administration of the drug, wherein when the phosphorylation level positively correlates with the cell growth inhibition rate, then the drug is a suitable drug candidate, wherein the biomarker is a DAPK protein having the sequence of SEQ ID NO: 2, wherein the serine 308 (Ser308) amino acid residue of the DAPK protein is phosphorylated.

According to embodiments of the present invention, the specimen is ascites, blood, urine, feces, sputum, mucosal cells, gastric fluid, bile, or detached cancer tissues collected after a surgery.

According to embodiments of the present invention, the candidate drug comprises sorafenib and GW5074.

In one aspect, the present invention also discloses the use of a biomarker for drug screening, wherein the biomarker is a DAPK protein having the sequence of SEQ ID NO: 2, wherein the serine 308 (Ser308) amino acid residue of the DAPK protein is phosphorylated,

According to the present invention, the candidate drug comprises sorafenib and GW5074.

According to the present invention, the use of a biomarker in screening a candidate drug further includes detecting a phosphorylation level of the biomarker of the specimen prior to the administration of the drug, and detecting the cell growth inhibition rate after the administration of the drug, wherein when the phosphorylation level positively correlates with the cell growth inhibition rate, then the drug is a suitable drug candidate.

The present disclosure therefore proposes a novel pharmaceutical composition for use in combination therapy for treating cancer, in which sorafenib, an antiangiogenic drug, is combined with GW5074, a c-Raf inhibitor. Neither of these two compounds, when administered alone at a low dosage (sorafenib at 5uM or GW5074 at 10uM), exhibits growth inhibition effects. However, the combination of these two drugs that are non-cytotoxic alone attains a cytotoxic effect, thereby reducing the side effect caused by sorafenib at its effective dose while effectively inhibiting cancer cell growth. The novel composition for use in combination therapy for treating cancer proposed by the present invention uncovers a unique molecular mechanism, wherein these two drugs destroy the protein complex of c-Raf and DAPK by binding to c-Raf, which in turn leads to cell apoptosis and can be used as new targets for future drug design. Studies performed by the present inventor establish that the composition for use in combination therapy for treating cancer is most effective in cancer cells with highly phosphorylated c-Raf s338, and is less effective to human cancer cells with lower DAPK expression or less phosphorylated DAPK-s308.

The drug for dissociating c-Raf and DAPK proteins or the drug screened by the present method is the combination of sorafenib and GW5074.

The term "pharmaceutically acceptable excipient" is used herein to refer to any physiologically inert or pharmacologically inactive substance known to a person skilled in the art that is physically or chemically compatible with either sorafenib or GW5074. Pharmaceutically acceptable excipients include, but are not limited to, polymers, resins, plasticizers, fillers, lubricants, diluents, binders, disintegrants, solvents, cosolvents, surfactants, preservatives, sweeteners, flavoring agents, pharmaceutically grade dyes or pigments, and viscosity agents.

In this application, the term "pharmaceutical composition" is a solid or liquid composition whose form, concentration and degree of purity are suitable for administration to a patient (such as a human or an animal patient) and may induce desired physiological changes after its administration. A pharmaceutical composition is typically sterile and / or non-pyrogenic.

As used herein, the term "combination" describes materials that combining combine two or more compounds and/or drugs (also called ingredients in this application). The term "combined" and "combining" have the same meaning.

The combination of two or more compounds /drugs in a composition can be physical or non-physical. The examples of a compound/drug composition that was bound physically include compositions (e.g. a single mixture) that contains two or more mixed compounds/drugs (e.g. in the same single dosage), a composition that contains two or more chemically/physically-linked compounds/drugs (e.g. by cross-linking, molecular agglomeration or binding to a common vehicle moiety), a composition that contains two or more compounds/drugs that are chemically or physically co-packaged (e.g. formulated in a liquid medium, particles (e.g. micron particles or nanoparticles) or materials on or inside the emulsion droplets, pharmaceutical kits, pharmaceutical packs, or patient packs, wherein two or more compounds/drugs are co-packaged or co-represented (e.g. a batch of dosage units).

The examples of a compound/drug composition with non-physical combination include: a material (e.g. non-single mixture) that contains at least one of the two or more compounds / drugs plus the instructions indicating that the physical combination of the two or more compounds / drugs is formed by the freshly made combination of at least one or more physical; a material (e.g. non-single mixture) that contains at least one of the two or more compounds /drugs plus instructions demonstrating a combination therapy utilizing two or more compounds / drugs; a material which contains at least one of the two or more compounds / drugs plus instructions for administration to a patient population, wherein the patient population has been treated with (or is taking) the other drug (others) of the two or more compounds / drugs; and a material that comprises at least one of the two or more compounds / drugs whose amount or form is specially formulated to be used in composition with the other (others) of the two or more compounds / drugs.

In this application, the term "combination therapy" is used herein to refer to a therapy using the composition containing two or more compounds/drugs (as defined above). Thus, in the present invention, "combination therapy," "combination," as well as "composition" of compounds/drugs are used to refer to compounds/drugs administered as part of the complete treatment. Consequently, the posology of each of the two or more compounds/drugs may be different, suggesting that each can be administered at the same or at different times. It is important to understand that the compounds/drugs of said composition may be given in order (e.g. before or after) or concurrently (e.g., at the same time), and may be formulated either in the same pharmaceutical mixture (together) or in different mixtures (separately). Furthermore, simultaneous administration in the same mixture is given as a single mixture, and simultaneous administration in different mixtures is not given as a single mixture. In addition, the posology of each of the two or more compounds/drugs in a combination therapy may also vary depending on modes of administration.

### BRIEF DESCRIPTION OF THE DRAWINGS

Figure 1 shows the growth inhibition of ACHN cells at 24 hours after the single-dose treatment of c-Raf inhibitors (sorafenib, GW5074, L779450 or PLX4720);
Figure 2 shows the growth inhibition of ACHN cells at 48 hours after the single-dose treatment of c-Raf inhibitors (sorafenib, GW5074, L779450 or PLX4720);
Figure 3 shows the growth inhibition of ACHN cells at 72 hours after the single-dose treatment of c-Raf inhibitors (sorafenib, GW5074, L779450 or PLX4720);
Figure 4 shows the growth inhibition of ACHN cells at 24, 48 and 72 hours after the combination therapy of various c-Raf inhibitors (Sor: sorafenib, GW: GW5074, L77: L779450, PLX: PLX4720);
Figure 5 shows the xenografted tumor volumes measured every 3 days for up to 21 days following sorafenib and GW5074 treatments administered singularly or in combination;
Figure 6 shows the photon signals collected from the immnunodeficient mice orthotopic xenografted with Luc-ACHN-LL cells following treatments of various drugs. To calculate the number of tumors distributed throughout the body, the total photons emitted from the entire body of each mouse was measured as well as quantified by Xenogen* living image software. The arrow indicates the time of initial drug administration;
Figure 7 shows the level of the apoptosis or necrosis of ACHN cells at 24 hours following treatments of DMSO (control group), 5 µM sorafenib and10 µM GW5074 or the combination therapy;
Figure 8 shows the phosphorylation level of pDAPKS308 and DAPK protein expression of ACHN cells at 24 hours following treatments of DMSO (control group), 5 µM sorafenib and 10 µM GW5074 or the combination therapy by immunoblotting;
Figure 9 shows the immunoprecipitation results of endogenous DAPK of ACHN cells at 24 hours after treatments of DMSO (control group), 5 µM sorafenib and 10 µM GW5074 or the combination therapy, followed by immunoblotting to stain other relevant proteins using specific antibodies;
Figure 10 shows the immunoprecipitation results of endogenous c-Raf of ACHN cells at 24 hours after treatments of DMSO (control group), 5 µM sorafenib and 10 µM GW5074 or the combination therapy, followed by immunoblotting to stain other relevant proteins using specific antibodies;
Figure 11 shows the growth inhibition of ACHN cells following the combination therapy in the presence or absence of PP2A inhibitor: cantharidin (C.A.) or okadaic acid (O.A.);
Figure 12 shows the growth inhibition of ACHN cells stably expressing the wild-type or c-Raf mutants at 24 hours following combination therapy. (**p* < 0.05, ***p* < 0.01 in comparison with the wild type c-Raf);
Figure 13 is a protein expression profile of ACHN cells expressing the flag-tagged-c-Raf or indicated mutants at 24 hours following the DMSO (Ctrl.) treatment or the combination therapy. Flag-c-Raf was immunoprecipitated from the cell lysates, followed by immunoblotting for indicated antibodies;
Figure 14 shows the growth inhibition of ACHN, 786-O and Rcc-Sut-002 cells transfected with the scrambled siRNA (Scr), DAPK siRNA1 (siDAPK-a), or DAPK siRNA2 (siDAPK-b) following combination therapy (upper panel). DAPK and α-catenin expression of the cell lysates were examined by immunoblotting;
Figure 15 shows the growth inhibition of HeLa cells expressing the V5-tagged-DAP and indicated mutants at 24 hours following the combination therapy;
Figure 16 shows the growth inhibition of MDA-MB231 cells expressing the V5-tagged-DAP and indicated mutants at 24 hours following the combination therapy;
Figure 17 shows the growth inhibition of various cancer cells or normal cells at 24 hours following treatments of 5 µM sorafenib.
Figure 18 shows the growth inhibition of various cancer cells or normal cells at 24 hours following treatments of 10 µM GW5074;
Figure 19 shows the growth inhibition of various cancer cells or normal cells at 24 hours following combination therapy of 5 µM sorafenib and 10 µM GW5074;
Figure 20 shows the phosphorylation level of pDAPK^{S308} of ACHN group in comparison with the control group; the expression of pDAPK^{S308} and DAPK was detected by immunoblotting;
Figure 21 is a regression plot demonstrating the correlation between the expression of pDAPK^{S308} and the growth inhibition of cells; R² = 0.4551, R = 0.6746, *p* = 0.00001;
Figure 22 shows the expression level of pDAPK^{S308} and DAPK in tumors (pT) or normal tissues (pN) of the same patient by immunoblotting;
Figure 23 shows the relative intensity of pDAPK^{S308} after normalization to GAPDH expression;
Figure 24 is the immnunohistochemical (IHC) analysis of pDAPK^{S308} expression of human normal and renal carcinoma tissues. Original magnification, x40 (upper panel), x100 (lower panel), scale bars is 100 µM;

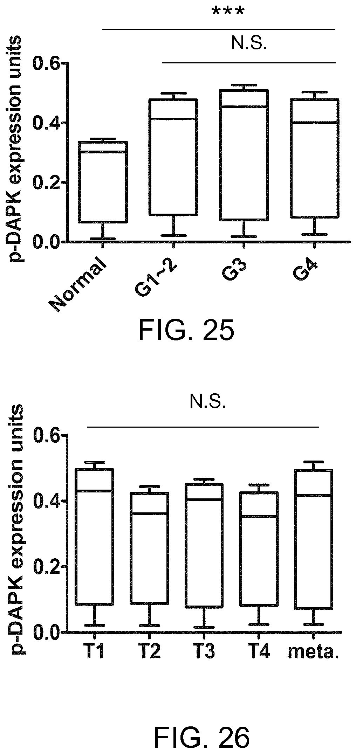

Figure 25 shows the pDAPK^{S308} expression of human normal and renal cancer tissues after the quantification using microarray; normal as well as different grades (G);
Figure 26 shows the pDAPK^{S308} expression of human normal and renal cancer tissues after quantification using microarrays; different stages (T) as well as metastasis of cancer cells (meta.);
Figure 27 is a computer simulation photo demonstrating the conformation of c-Raf kinase after the interaction with sorafenib and GW5074; Green represents GW5074 and magenta represents sorafenib.

### DETAILED DESCRIPTION OF THE PREFERRED EMBODIMENT

The present invention will now be described more specifically with reference to the following examples, which are provided for the purpose of demonstration rather than limitation.

### Example 1 - Combination therapy of Sorafenib and GW5074- cell testing

Human renal carcinoma (ACHN) cells were cultured in Eagle MEM (Minimum Essential Medium) with 10% FBS (Fetal Bovine Serum) and 1% penicillin/streptomycin. Sulforhodamine B (SRB) is a negative protein with a sulfonic acid group and binds to basic amino acids of intracellular proteins in weak acidic conditions. The SRB protein was extracted from cells using weak alkaline solution and then subjected to absorbance measurement. The amount of intracellular proteins, which is an indicator for cell survival, can be calculated from the amount of SRB. ACHN or A498 cells were treated singularly with 2.5 µM sorafenib, 5 µM sorafenib, 10 µM sorafenib, GW5074, L779450 or PLX4720 for 24, 48 and 72 hours, followed by SRB assay to assess cell survival. Alternatively, ACHN cells were pre-treated with 10 µM GW5074, 10 µM L779450 or 10 µM PLX4720 for 30 minutes prior to 5 µM sorafenib treatment for additional 24, 48 and 72 hours, followed by SRB assay to assess growth inhibition.

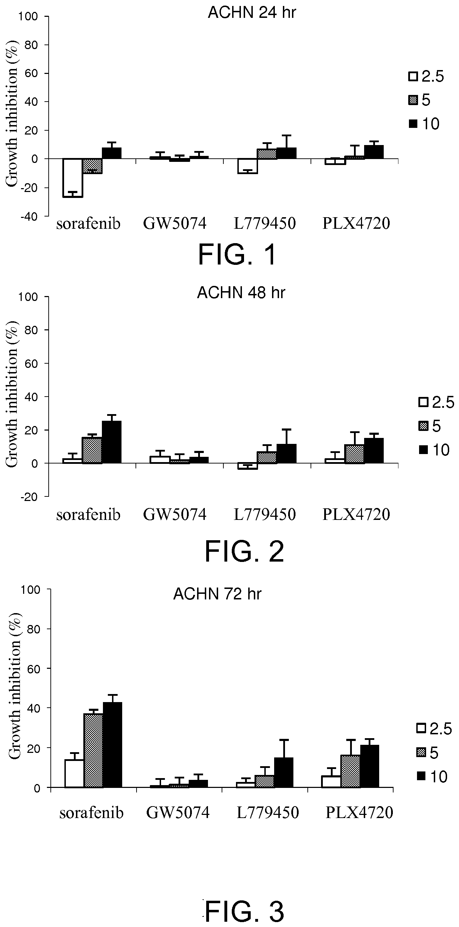

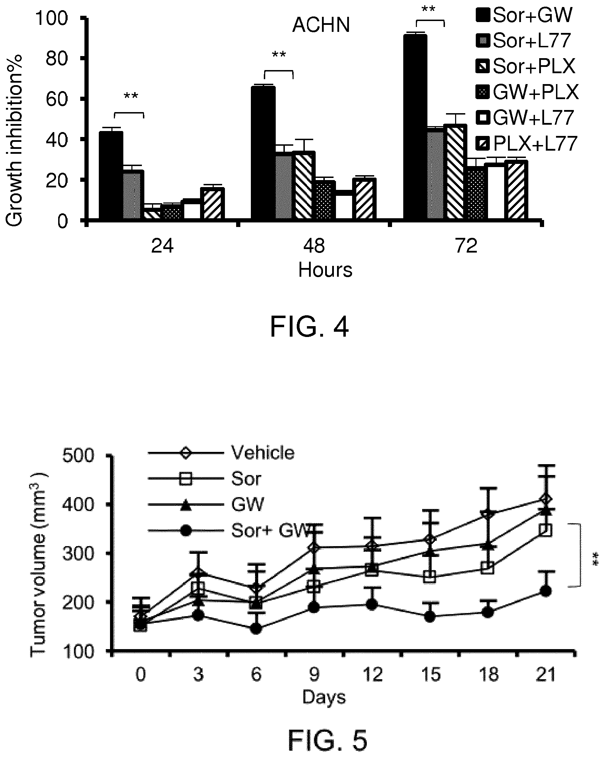

Based on these results, the combination of c-Raf inhibitors comprising sorafenib and GW5074 showed no growth inhibition of cells following the single low-dose treatment for 24 hours (5 µM sorafenib or 10 µM GW5074) (Figures 1 to 3, mean±S.D., n=4). However, the combination of these two drugs not only induced the cytotoxicity which was not observed in the sole treatment of either drug originally, but successfully reduced side effects caused by the effective dosage of sorafenib and inhibited the cancer cell growth, suggesting a synergistic effect (Figure 4, mean±S.D., ***P* < 0.01, n=4).

### Example 2 - Combination therapy of sorafenib and GW5074- xenograft

Six-week old immnunodeficient male mice (BALB/cAnN.Cg-Foxn1^{nu}/CrlNarl) with an average weight of 20 grams were xenografted with 1x10⁷ ACHN cells to the right by intraperitoneal injection (i.p.). The mice were maintained in a specific pathogen-free (SPF) environment. The size of tumor was measured using a digital caliper twice a week and tumor volume was calculated by the equation of length^{∗}width^{∗}height^{∗}0.5. The drug administration which included 5 mg/kg sorafenib by oral gavage and subcutaneous injection of 10 mg/kg GW5074 once a day for three weeks was initiated when the tumor volume was > 100mm³, and the size of tumor was measured every three days. A total of four groups were included in the experiment: a control group which received only vehicle (DMSO), and test groups administered with 5 mg/kg sorafenib, 25 mg/kg GW5074, or the combination therapy of 5 mg/kg sorafenib and 25 mg/kg GW5074, respectively, with 8 mice in each group.

The results are shown in Figure 5. The treatment of either 5 µM sorafenib or 10 µM GW5074 alone exhibited nearly no inhibition effect. In contrast, the combination therapy of 5 µM sorafenib and 10 µM GW5074 demonstrated a significant growth inhibition of ACHN cells (t-test, mean±SD, ***P*<0.01, n-8).

### Example 3 - Combination therapy of sorafenib and GW5074 - the orthotopic model

To simulate clinical phenomenon, we established an orthotopic spontaneous animal model for studying metastasis of renal carcinoma. First, ACHN cells were transfected with the luciferase gene and the resultamt Luc-ACHN transfectants (stable transfectants which express the luciferase) with different expression levels were then selected for in vivo culture in mice by subcutaneous injection. Six-week old and shaved mice were subcutaneously injected with 1×10⁷ Luc-ACHN cells In 0.1 ml PBS. Two months after the injection, the mice were sacrificed and their kidneys, livers, regional lymph nodes as well as other organs were collected for assessing tumor cells that were potentially metastatic, which was confirmed by hematoxylin-eosin (H&E) staining. Tumor cell lines that are highly metastatic were dissected in 1 ml PBS into small pieces aseptically and then cultured in MEM media containing 10% FBS (fetal bovine serum) and 1% penicillin/streptomycin following centrifugation. A few days later, monoclonal cell lines were first treated with the trypsin to dissociate the cells and then cultured in vitro. The cells obtained from liver tumor tissues were named ACHN-L. The metastatic potential of various monoclonal cell lines collected from different organs was further analyzed by xenografting these tumor cells to the left kidneys of mice. The xenografted mice were sacrificed and examined 8 weeks after the injection of tumor cells. The tumor growth observed in each organ was investigated through visual examination and histology procedures. The metastatic cells found in the liver were dissected into small pieces aseptically and cultured in vitro. The cells obtained from liver tumor tissues were named ACHN-L (subcutaneous injection) cells and the cells collected from the metastatic lesions after injection of ACHN-L cells to the left kidneys were called ACHN-LL cells. The same procedure using orthotopic xenograft of ACHN-LL cells and liver metastatic cells was repeated twice to select for highly metastatic tumors. The cancer cell line (Luc-ACHN-LL), which is highly metastatic and causes high mortality, was selected. Immunodeficient male mice was injected with 3×10⁵ Luc-ACHN-LL cells in 50 µL PBS at the right renal capsule. The in vivo luciferase activity was examined every day for up to three weeks so as to assure that there were no leakage of cancer cells at the kidneys immediately after the injection and to monitor the metastasis of these cancer cells. Two to five minutes before utilizing the IVIS Xenogene system, 75 mg/kg D-Luciferin (Xenogen) in PBS was injected to the retro-orbital sinus of each mouse. Two weeks after the injection of Luc-ACHN-LL cells, IVIS Xenogene system was used to monitor transfer of biological fluorescent Images and to calculate the intensities of the photon signals (photons/s/cm²/steradian). A total of five groups were included in the study: a control group which received only vehicle, and four test groups which respectively received 10 mg/kg sorafenib, 25 mg/kg GW5074, a combination therapy of 10 mg/kg sorafenib and 25 mg/kg GW5074 (the animals were fed with Sor 30 min post i.p. injection of GW), or 60 mg/kg sorafenib.

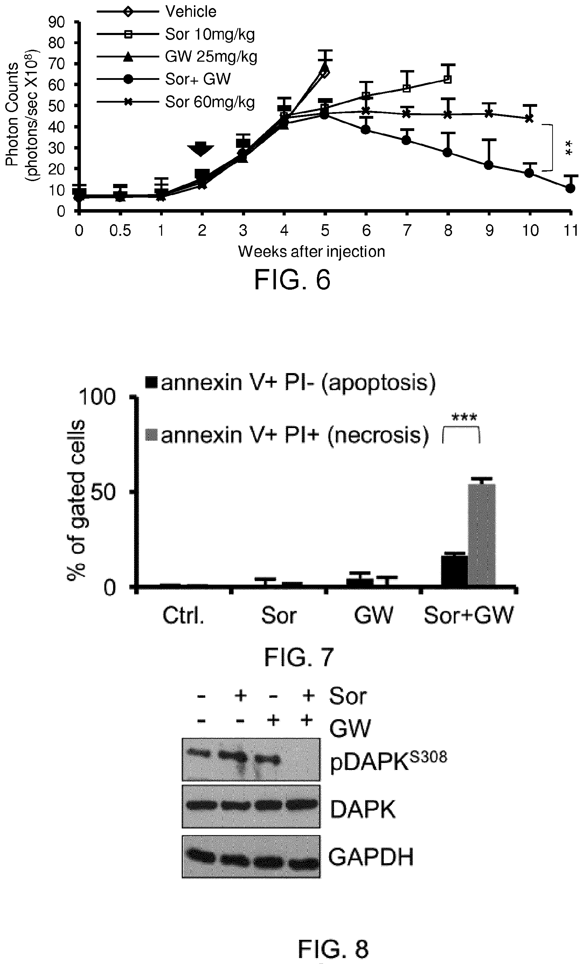

As shown in the results, the mice of the control group, 10 mg/kg sorafenib group, and 25 mg/kg GW5074 group died within 5, 8, and 5 weeks following injection of cancer cells, respectively. Likewise, loss of weight was also observed in the high-dose test group which received 60 mg/kg sorafenib, followed by death within 10 weeks. Surprisingly, only mice in the group which received low-dose combination therapy of 10 mg/kg sorafenib and 25 mg/kg GW5074 showed the inhibition of both tumor sizes and metastasis of tumors as well as the prolonged survival period in comparison with other groups. Figure 6 shows the IVIS images and quantification of the photon intensity, mean±SD, ***P*<0.01, n=4. In addition, the body weights as well as the energy of the mice that received combination therapy were similar to those observed in normal mice. The tumor cells collected from the mice that received the combination therapy also showed profound cell necrosis in comparison with other groups.

In summary, the combination therapy of low-dose sorafenib and GW5074 is effective in growth inhibition of cancer cells in vitro. Also, most importantly, the efficacy of combination therapy can be examined in vivo in the condition that simulates clinical metastasis of cancer cells.

### Example 4

The combination therapy of sorafenib and GW5074 induces cell necrosis through the dephosphorylation of the death-associated DAPK at serine 308 followed by dissociation from proto-oncogene c-Raf. Cells were cultured as described in example 1, washed with phosphate buffered saline (PBS), and stained with the annexin V-FITC and propidium iodide (PI) for 15 minutes. The presence of fluorescent Annexin V as well as propidium iodide (PI) was detected by flow cytometry so as to determine whether the cell death is caused by apoptosis or necrosis. The expression of c-Raf and DAPK as well as the phosphorylation level of DAPK were examined by Western blot and immunoprecipitation using antibodies. Anti-DAPK antibodies or non-immune rabbit IgG (IP: immunoglobulin) were included as negative controls, and cell survival was measured by SRB assay, as shown In example 1. In this experiment, ACHN cells were treated separately with DMSO (as a control group), 5 µM sorafenib, 10 µM GW5074, or the combination therapy of 5 µM sorafenib and 10 µM GW5074 for 24 hours.

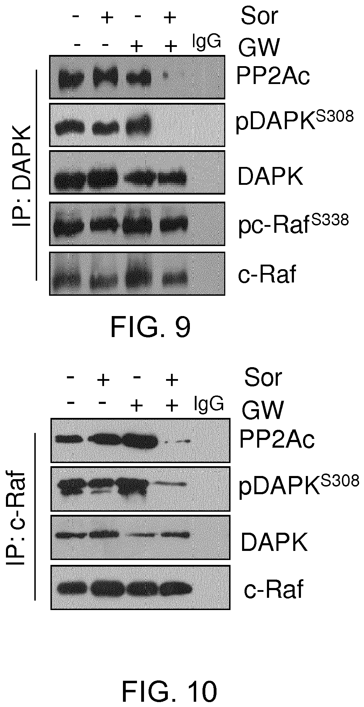

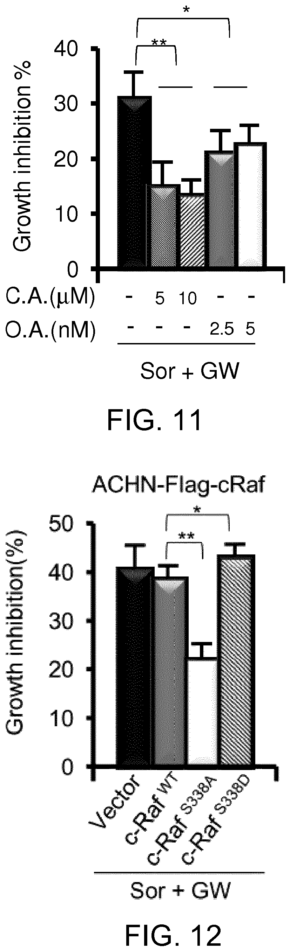

The results indicated that the average cell necrosis rate of ACHN cells treated with DMSO, the monotherapy of 5 µM sorafenib, and monotherapy of 10 µM GW5074 was 0.37, 0.77, and 1.35%, respectively. However, cells which received the combination therapy showed significant increase of necrosis, up to 53.95% (Figure 7, t-test, mean±SD, ****P*<0.001, n=4). A significant decrease in phosphate groups of S308 due to dephosphorylation of pDAPK^{S308} by PP2A in tumor cells was only observed in cells treated with combination therapy (Figure 8). Results from the immunoprecipitation further suggested c-Raf and DAPK, as well as PP2A, formed a complex. Combination therapy reduced the interactions among PP2A, DAPK and c-Raf, which subsequently decreased pDAP^{S308} (Figures 9 and 10). For the test group which received the combination therapy, various concentrations of PP2A inhibitors (Cantharidin acid, C.A. and Okadaic acid, O.A.) were effective in the inhibition of dephosphorylation of pDAPK^{S308} and growth of ACHN cells only when provided 30 minutes prior to the administration of the combination drugs (Figure 11, * *P* <0.05, ** *P* <0.01, n = 4).

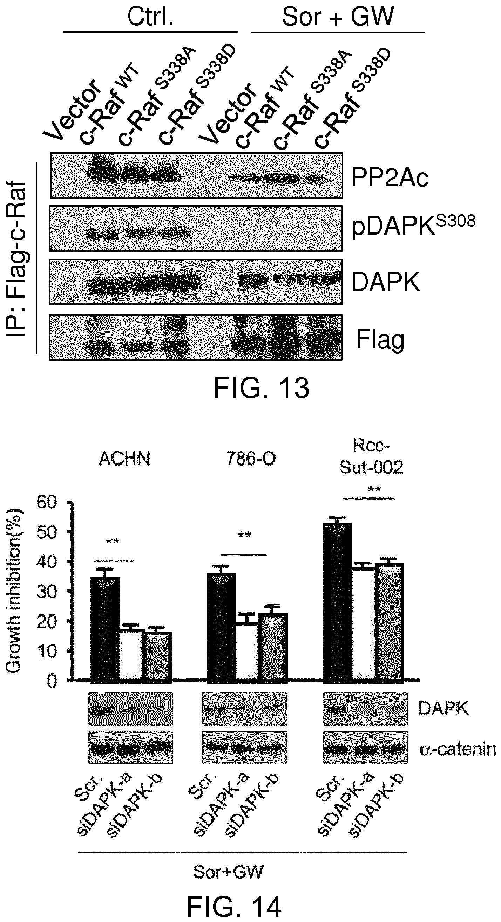

Next, the effects of the combination therapy on the efficacy of pc-Raf^{S338}. Reduction of pDAPK^{S308} was noted as being most significant when ACHN cells were transfected with c-Raf^{S338D} (simulation of phosphorylating S338). Moreover, c-Raf^{S338A} (simulation of dephosphorylating S338) not only weakened the dephosphorylation of DAPK S308, but also reduced the growth inhibition of ACHN cells under the combination therapy (Figure 12, * *P* <0.05, ** *P* <0.01, n=3). In addition, Immunoprecipitation results suggested that c-Raf^{S338D} (simulation of phosphorylating S338) apparently lost its interaction with PP2A in comparison with c-Raf^{WT} (wilt type) and c-Raf^{S338A} (simulation of dephosphorylating S338) (Figure 13). Therefore, the combination therapy not only increases pc-Raf^{S338}, but also facilitates the dephosphorylation of pDAPK^{S308} by PP2A and enhances the cell necrosis.

The above-mentioned example reveals that combination therapy of sorafenib and GW5074 induces the cell necrosis through the dephosphorylating serine 308 of the death-associated protein kinase (DAPK) followed by the dissociation from the proto-oncogene c-Raf. The disassembly of c-Raf and DAPK can also be used as the target for future drug design, since the dissociation of c-Raf from DAPK in cells leads to the initiation of cell necrosis process that kills cells effectively.

### Example 5 - Methods of drug screening using in vitro biomarkers

Previous studies showed that the dephosphorylation of DAPK at serine 308 can be used as a predictive biomarker for anticancer effects of drugs on cancer cells. The methods for cell culture as well as the detection for examining growth inhibition are as described in example 1. ACHN, 786-O and Rcc-Sut- 002 (drug-resistant cancer cells) cell lines were treated with siRNA of type a and type b DAPK (siDAPK-a, sense strand: 5'-CAAGAAACGUUAGCAAAUGUU-3' [SEQ ID No:3] and antisense strand: 5'-CAUUUGCUAACGUUUCUUGUU-3' [SEQ ID No:4]; siDAPK-b, sense strand: 5'-GGUCAAGGAUCCAAAGAAGUU-3' [SEQ ID No:5] and antisense strand 5'-CUUCUUUGGAUCCUUGACCUU-3' [SEQ ID No.6]). Each cell line was analyzed by siDAPK-a, siDAPK-b and a control (Scr). Growth inhibition of cells was examined following combination therapy of 5 µM sorafenib and 10 µM GW5074 for 24 hours. On the other hand, HeLa cells and MDA-MB-231 cells were transfected with the control empty vector (vector), WT (wild type), DAPK^{S308D}, DAPK^{S308A} or DAPK^{K42A} (inactivated protein kinase), and the growth inhibition of the transfected cells was examined following the combination therapy of 5 µM sorafenib and 10 µM GW5074 for 24 hours.

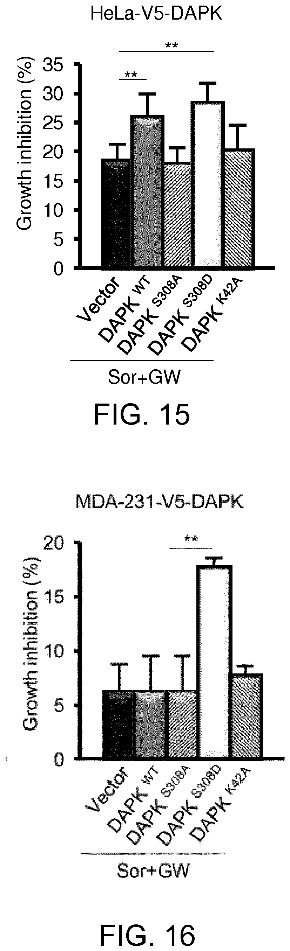

The results indicated that the DAPK protein is indispensible for the cytotoxicity induced by the combination therapy of sorafenib and GW5074. In ACHN, 786-O and RCC-SUT-002 cells, the growth Inhibition resulting from two siRNAs capable of inhibiting the DAPK expression was reduced by around 60% (Figure 14). Furthermore, the phosphorylation of DAPK at S308 is highly associated with the cytotoxicity induced by the combination therapy. Overexpression of DAPK^{WT} and DAPK^{S308D} in HeLa cells which usually express higher pDAPK^{S308} notably increased the cytotoxicity after receiving the combination therapy, whereas the cell death was not enhanced in DAPK^{K42A} (inactivated DAP kinase) and DAPK^{S308A} (simulation of non-phosphorylation of S308) following the combination therapy. Only DAPK^{S308D} was found to increase the growth inhibition in MDA-MB-231 breast cancer cells which usually express lower pDAPK^{S308} after receiving the combination therapy. The rest, including DAPK^{WT}, DAPK^{S308A}, and DAPK^{K42A}, showed no increase in the growth inhibition (Figures 15 and 16, mean±SD, ****p < 0.01, n = 3). According to the results, it is not the DAPK protein itself, but the S308 phosphorylation of DAPK which plays a key role in inducing the cytotoxicity when treated with the combination therapy of sorafenib and GW5074. This is because the dephosphorylation of S308 is not effective under the combination therapy even with a higher DAPK expression. The cytotoxicity effect induced by the combination therapy must go through activated DAPK. Therefore, the combination therapy of sorafenib and GW5074 is only effective in the presence of pDAPK^{S308} in cancer cells.

Phosphorylation of c-Raf protein can be used as a biomarker for predicting the anticancer effect of drugs on cancer cells. Reduction of pDAPK^{S308} is most evident in ACHN cells transfected with c-Raf^{S338D} (simulating phosphorylation of S388). Not only does c-Raf^{S338A} (simulating dephosphorylation of S388) reduce the dephosphorylation of DAPK S308, but also decreases the growth inhibition of ACHN cells under the combination therapy (Figure 12, * *P* <0.05, ** *P* <0.01, n=3). Thus, the S338 phosphorylation of c-Raf is beneficial for the combination therapy due to better treatment effects, and c-Raf S338D (simulating phosphorylation of S388) has a better effect in comparison with c-Raf S338A (simulating dephosphorylation of S388).

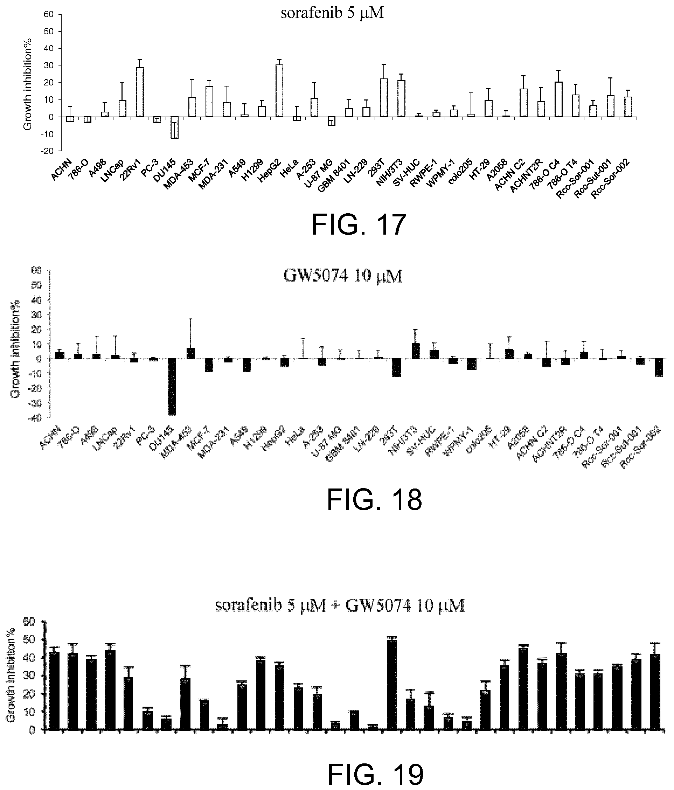

In addition, a number of cancer cells as well as normal cells were examined. It was found that the combination therapy caused only limited cytotoxicity in the above-mentioned cells due to the low S308 phosphorylation of DAPK in normal fibroblasts and epithelial cells. Nonetheless, the growth inhibition caused by the combination therapy among various tumor cells is positively correlated with the level of S308 phosphorylation of DAPK (Figures 17, 18, 19, 20, and 21, mean±SD, ***p* < 0.01, n = 3). It was further investigated as to whether the combination therapy has an inhibition effect on drug-resistant cancer cells using sorafenib-resistant cancer cells obtained from clinical cases (RCC-Sut-001 ^{,} RCC-Sut-002 , RCC-Sor-001) and animal models (786- OT4, ACHN-T2R). All drug-resistant cancer cells that have highly phosphorylated DAPK S308 were significantly inhibited under the combination therapy. Moreover, HT29 and A2058 cancer cell lines that are both Raf inhibitors-resistant showed high S308 phosphorylation of DAPK as well, and the combination therapy demonstrated significant inhibition as well as synergistic effect (Figures 17, 18, 19 and 20, and Table below). Additionally, because the S308 phosphorylation of DAPK in normal cells are relatively low, the growth inhibition of cancer cells induced by the combination therapy of sorafenib and GW5074 are therefore selective and cause no toxicity in normal cells.

| | | | |
|---|---|---|---|
| 1 | ACHN | Human renal cell adenocarcinoma | |
| 2 | 786-O | Human renal cell adenocarcinoma | |
| 3 | A498 | Human renal cell adenocarcinoma | |
| 4 | LNCap | Prostate carcinoma | |
| 5 | 22RV1 | Prostate carcinoma | |
| 6 | PC-3 | Prostate carcinoma | |
| 7 | DU-145 | Prostate carcinoma | |
| 8 | MDA-453 | Breast adenocarcinoma | |
| 9 | MCF-7 | Breast adenocarcinoma | |
| 10 | MDA-231 | Breast adenocarcinoma | |
| 11 | A549 | Human lung carcinoma | |
| 12 | H1299 | Non-small cell lung cancer | |
| 13 | HepG2 | Hepatocellular carcinoma | |
| 14 | HeLa | Cervix carcinoma | |
| 15 | A253 | Submaxillary salivary gland carcinoma (oral carcinoma) | |
| 16 | U87 | Glioblastoma ; astrocytoma | |
| 17 | GBM8401 | Brain malignant glioma | |
| 18 | LN229 | Glioblastoma | |
| 19 | 293T | Kidney epithelial | |
| 20 | 3T3 | Embryo fibroblast | |
| 21 | SV-HOC | Uroepithelium epithelial | |
| 22 | RWPE1 | Prostate normal epithelial cell | |
| 23 | WPMY1 | Prostate normal epithelial cell | |
| 24 | Colo-205 | Colorectal adenocarcinoma | |
| 25 | HT-29 | Colorectal adenocarcinoma | |
| 26 | A2058 | Melanoma | B-Raf mutation |
| 27 | ACHNC2 | Drug-resistant renal cell adenocarcinoma cell | Acquired resistant |
| 28 | ACHNT2R | Drug-resistant renal cell adenocarcinoma cell | Acquired resistant |
| 29 | 786-OC4 | Drug-resistant renal cell adenocarcinoma cell | Acquired resistant |
| 30 | 786-OT4 | Drug-resistant renal cell adenocarcinoma cell | Acquired resistant |
| 31 | RCC-Sor-001 | Drug-resistant renal cell adenocarcinoma cell | Acquired resistant |
| 32 | RCC-Sut-001 | Drug-resistant renal cell adenocarcinoma cell | Acquired resistant |
| 33 | RCC-Sut-002 | Drug-resistant renal cell adenocarcinoma cell | Acquired resistant |

In view of the data provided in Table 1, above, c-Raf and DAPK are found in the cytoplasm and the mitochondria, and the combination therapy lead to the relocation of DAPK between the cytoplasm and the mitochondria, along with the dephosphorylation of pDAPK^{S308} by PP2A. The dephosphorylated DAPK decreases its interaction with c-Raf in the cytoplasm. In addition, only DAPK^{S308D} can be induced to be translocated from the mitochondria to the cytoplasm of MDA-MB-231 under the combination therapy. This results in the production of ROS and the low phosphorylation of pDAPK^{S308}. However, the reduction of both c-Raf and the phosphorylation of its S338 induced by the combination therapy in the cytoplasm and the mitochondria was found only in cancer cells with highly phosphorylated pDAPK^{S308} and not in cancer cells with low pDAPK^{S308},

### Example 6

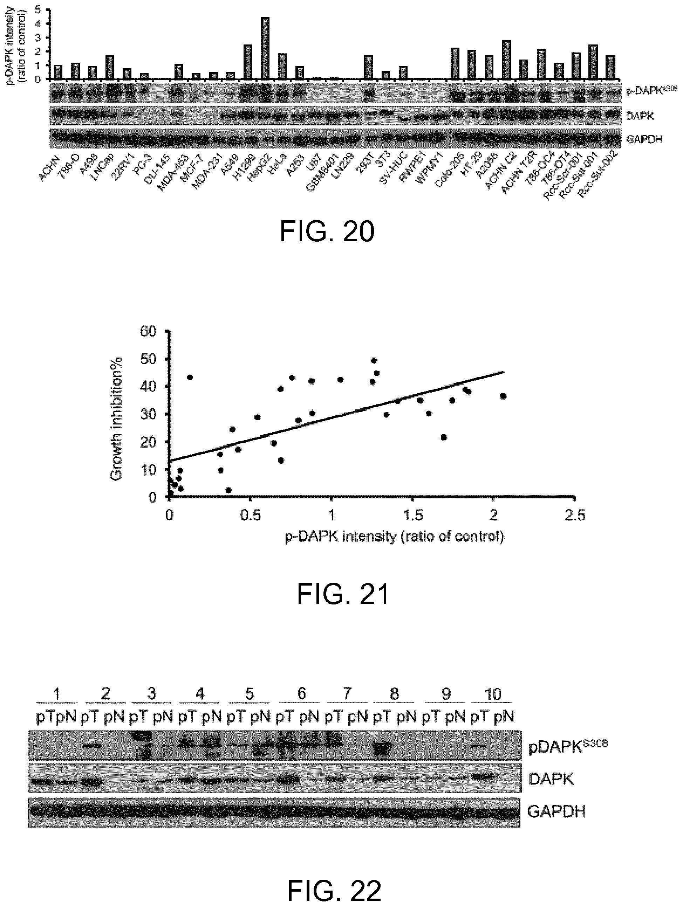

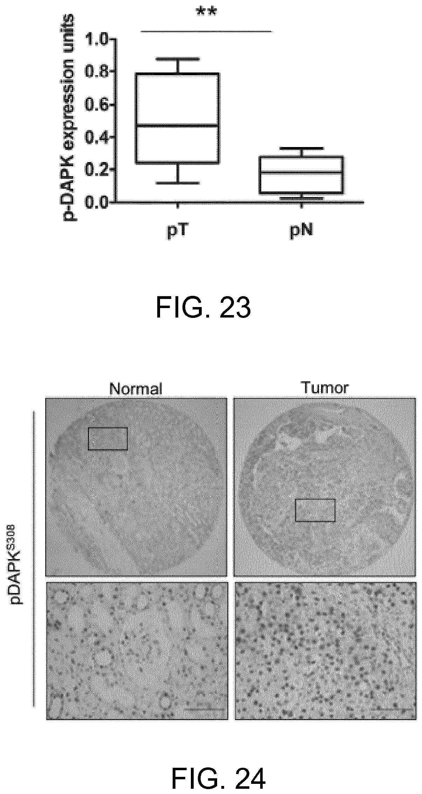

The tumor samples and normal tissue samples collected from 20 patients with renal cell carcinoma were further investigated. Based on the Western blot results, 16 out of 20 samples showed elevated phosphorylation of DAPK at S308 in cancer cells in comparison with normal tissue samples (Figures 22 and 23 (pDAPK^{S308}/GAPDH, mean± S.D., ***p* <0.005)). Immnunohistochemical (IHC) analysis also demonstrated that S308 phosphorylation of DAPK was higher in 181 human renal carcinoma samples in comparison with normal kidney tissues (Figure 24). On the other hand, the S308 phosphorylation of DAPK showed no significant differences between different grades (G) or stages (T) of the cancer using the tissue microarray (TMA) and the semi-quantitative analysis. The results suggested that pDAPK^{S308} is not only a factor for determining prognosis, but also a predictive biomarker of combination therapy for treating kidney cancers.

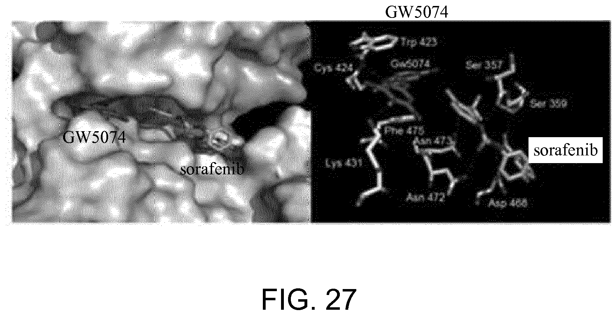

According to the results, a computer simulation experiment was then conducted to assess the crystal structures of various c-Raf inhibitors bound with c-Raf. The maximal energy was expected to be produced by the binding of inhibitors to the structural region of c-Raf kinase when the combination of GW5074 and sorafenib binds to c-Raf (-182 kcal/mole, Table below). GW5074 at the front end binds with c-Raf and produces a deeper hydrophobic pocket for binding through ile355, Val363, Ala373, Leu406, Trp423 and Phe47. Under the circumstances, more regions in the hydrophobic pocket will be occupied by GW5074 and sorafenib (as shown in Figure 27).

| Inhibitor | | Binding energy (kcal/mole) |
|---|---|---|
| Name | Structu re | |
| sorafenib | 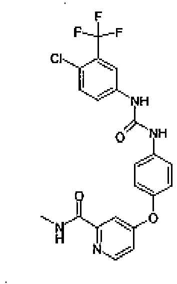 | -82 |
| + L779450 | | -134 |
| + GW5074 | | -125 |
| + PLX44720 | | -113 |
| GW5074 | 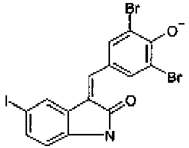 | -128 |
| + Sorafenib | | -182 |
| PLX44720 | 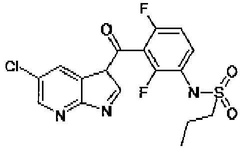 | -77 |
| + sorafenib | | -140 |
| L779450 | 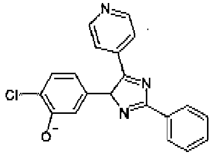 | -60 |
| + sorafenib | | -125 |

The present invention discloses a novel pharmaceutical composition for use in treating cancer, wherein such composition comprises sorafenib and GW5074. In use, such composition is not toxic to normal cells due to selective inhibition on cancer cells. Therefore, the use of such a composition is safe and very promising for future applications. The efficacy of such composition for use in treating cancer was verified by using pDAPKS308 as a predictive biomarker. The research of the present invention overcomes the obstacles faced by current studies of compounds for unse in cancer therapies and meets the requirements of an ideal model for use in preclinical treatments.

## Claims

1. A pharmaceutical composition for use in treating cancer, comprising sorafenib or a pharmaceutically acceptable salt thereof and 3-(3,5-Dibromo-4-hydroxy-benzylidene)-5-iodo-1,3-dihydro-indol-2-one or a pharmaceutically acceptable salt thereof.

2. The pharmaceutical composition for use in treating cancer according to claim 1, wherein the sorafenib and 3-(3,5-Dibromo-4-hydroxy-benzylidene)-5-iodo-1,3-dihydro-indol-2-one are administered separately, concurrently or sequentially.

3. The pharmaceutical composition for use in treating cancer according to claim 1, wherein the cancer is renal cell carcinoma, prostate cancer, breast cancer, lung cancer, cervical carcinoma, oral cancer, glioma, urothelial cell carcinoma, or melanoma.

4. The pharmaceutical composition for use in treating cancer according to claim 1, further comprising a pharmaceutically acceptable vehicle.

5. The pharmaceutical composition for use in treating cancer according to claim 4, wherein the vehicles is selected from the group consisting of, excipients, diluents, thickeners, fillers, binders, disintegrants, lubricants, oil or non-oil agents, surfactants, suspending agents, gelling agents, adjuvants, preservatives, antioxidants, stabilizers, and coloring agents.

6. The pharmaceutical composition for use in treating cancer according to claim 1, wherein the pharmaceutical composition is administered via oral administration, immersion, injection, topical applications, or patch administration.

7. The pharmaceutical composition for use in treating cancer according to claim 1, wherein the 3-(3,5-Dibromo-4-hydroxy-benzylidene)-5-iodo-1,3-dihydro-indol-2-one alters the conformation of a c-Raf protein having the sequence of SEQ ID NO: 1 upon binding thereto, thereby increasing the binding affinity of the sorafenib toward the c-Raf protein so as to dissociate c-Raf from the c-Raf/DAPK protein complex.

8. A combination of compounds for use in treating cancer, wherein the combination comprises sorafenib and 3-(3,5-Dibromo-4-hydroxy-benzylidene)-5-iodo-1,3-dihydro-indol-2-one, and wherein the combination of compounds dissociate a protein complex in cells, wherein the protein complex is a c-Raf/DAPK protein complex, the c-Raf protein having the sequence of SEQ ID NO: 1 and the DAPK protein having the sequence of SEQ ID NO: 2.

9. A method for screening a candidate drug using a biomarker, comprising the steps of, providing a specimen;
detecting a phosphorylation level of the biomarker in the specimen before the administration of a test drug, and detecting a cell growth inhibition rate of the test drug on the specimen, and when the phosphorylation level of the biomarker positively correlates with the cell growth inhibition rate, determining that the test drug is the candidate drug, wherein,
the biomarker is a DAPK protein having the sequence of SEQ ID NO: 2, wherein the serine 308 (Ser308) amino acid residue of the DAPK protein is phosphorylated.

10. The method of claim 9, wherein the specimen is ascites, blood, urine, feces, sputum, mucosal cells, gastric fluid, bile, or detached cancer tissues collected after a surgery.

11. The method of claim 9, wherein the candidate drug comprises sorafenib and 3-(3,5-Dibromo-4-hydroxy-benzylidene)-5-iodo-1,3-dihydro-indol-2-one.

12. Use of a biomarker in screening a candidate drug, wherein the biomarker is a DAPK protein having the sequence of SEQ ID NO: 2, wherein the serine 308 (Ser308) amino acid residue of the DAPK protein is phosphorylated.

13. The use of claim 12, wherein the candidate drug comprises sorafenib and 3-(3,5-Dibromo-4-hydroxy-benzylidene)-5-iodo-1,3-dihydro-indol-2-one.

14. The use of claim 12, further comprising detecting a phosphorylation level of the biomarker before the administration of a test drug, and detecting a cell growth inhibition rate of the test drug, and when the phosphorylation level of the biomarker positively correlates with the cell growth inhibition rate, determining that the test drug is the candidate drug.

## Patentansprüche

1. Pharmazeutische Zusammensetzung zur Verwendung bei der Behandlung von Krebs, umfassend Sorafenib oder ein pharmazeutisch annehmbares Salz davon und 3-(3,5-Dibrom-4-hydroxy-benzyliden}-5-iod-1,3-dihydro-indol-2-on oder ein pharmazeutisch verträgliches Salz davon.

2. Pharmazeutische Zusammensetzung zur Verwendung bei der Behandlung von Krebs nach Anspruch 1, wobei das Sorafenib und 3-(3,5-Dibrom-4-hydroxy-benzyliden)-5-iod-1,3-dihydro-indol-2-on getrennt, gleichzeitig oder nacheinander verabreicht werden.

3. Pharmazeutische Zusammensetzung zur Verwendung bei der Behandlung von Krebs nach Anspruch 1, wobei der Krebs ein Nierenzellkarzinom, Prostatakrebs, Brustkrebs, Lungenkrebs, Gebärmutterhalskrebs, Mundkrebs, Gliom, Urothelzellkarzinom oder Melanom ist.

4. Pharmazeutische Zusammensetzung zur Verwendung bei der Behandlung von Krebs nach Anspruch 1, ferner umfassend einen pharmazeutisch annehmbaren Träger.

5. Pharmazeutische Zusammensetzung zur Verwendung bei der Behandlung von Krebs nach Anspruch 4, wobei die Träger aus der Gruppe ausgewählt sind, die aus Hilfsstoffen, Verdünnungsmitteln, Verdickungsmitteln, Füllstoffen, Bindemitteln, Sprengmitteln, Gleitmitteln, öligen oder nicht öligen Mitteln, Tensiden, Suspendiermitteln, Geliermitteln, Adjuvanzien, Konservierungsmitteln, Antioxidanzien, Stabilisatoren und Färbemitteln besteht.

6. Pharmazeutische Zusammensetzung zur Verwendung bei der Behandlung von Krebs nach Anspruch 1, wobei die pharmazeutische Zusammensetzung über orale Verabreichung, Immersion, Injektion, topische Anwendungen oder Pflasterverabreichung verabreicht wird.

7. Pharmazeutische Zusammensetzung zur Verwendung bei der Behandlung von Krebs nach Anspruch 1, wobei das 3-(3,5-Dibrom-4-hydroxy-benzyliden)-5-iod-1,3-dihydro-indol-2-on die Konformation eines c-Raf-Proteins mit der Sequenz der SEQ ID NO: 1 bei der Bindung daran alteriert, wodurch die Bindungsaffinität des Sorafenibs zum c-Raf-Protein erhöht wird, um c-Raf vom c-Raf/DAPK-Proteinkomplex zu dissoziieren.

8. Kombination von Verbindungen zur Verwendung bei der Behandlung von Krebs, wobei die Kombination Sorafenib und 3-(3,5-Dibrom-4-hydroxy-benzyliden)-5-iod-1,3-dihydro-indol-2-on umfasst und wobei die Kombination von Verbindungen einen Proteinkomplex in Zellen dissoziiert, wobei der Proteinkomplex ein c-Raf/DAPK-Proteinkomplex ist, wobei das c-Raf-Protein die Sequenz der SEQ ID NO: 1 aufweist und das DAPK-Protein die Sequenz der SEQ ID NO: 2 aufweist.

9. Verfahren zum Screening eines Kandidatenarzneimittels unter Verwendung eines Biomarkers, umfassend die folgenden Schritte:
1. Bereitstellen einer Probe;
2. Nachweisen eines Phosphorylierungsniveaus des Biomarkers in der Probe vor der Verabreichung eines Testarzneimittels und Nachweisen einer Hemmrate des Zellwachstums des Testarzneimittels in der Probe, und wenn das Phosphorylierungsniveau des Biomarkers positiv mit der Hemmrate des Zellwachstums korreliert, Bestimmen, dass das Testarzneimittel das Kandidatenarzneimittel ist, wobei
3. der Biomarker ein DAPK-Protein mit der Sequenz der SEQ ID NO: 2 ist, wobei der Serin 308(Ser308)-Aminosäurerest des DAPK-Proteins phosphoryliert ist.

10. Verfahren nach Anspruch 9, wobei die Probe Aszites, Blut, Urin, Stuhl, Sputum, Schleimhautzellen, Magenflüssigkeit, Galle oder abgelöstes Krebsgewebe, das nach einer Operation gewonnen wurde, ist.

11. Verfahren nach Anspruch 9, wobei das Kandidatenarzneimittel Sorafenib und 3-(3,5-Dibrom-4-hydroxy-benzyliden)-5-iod-1,3-dihydro-indol-2-on umfasst.

12. Verwendung eines Biomarkers beim Screening eines Kandidatenarzneimittels, wobei der Biomarker ein DAPK-Protein mit der Sequenz der SEQ ID NO: 2 ist, wobei der Serin 308(Ser308)-Aminosäurerest des DAPK-Proteins phosphoryliert ist.

13. Verwendung nach Anspruch 12, wobei das Kandidatenarzneimittel Sorafenib und 3-(3,5-Dibrom-4-hydroxy-benzyliden)-5-iod-1,3-dihydro-indol-2-on umfasst.

14. Verwendung nach Anspruch 12, ferner umfassend das Nachweisen eines Phosphorylierungsniveaus des Biomarkers vor der Verabreichung eines Testarzneimittels und Nachweisen einer Hemmrate des Zellwachstums des Testarzneimittels, und wenn das Phosphorylierungsniveau des Biomarkers positiv mit der Hemmrate des Zellwachstums korreliert, Bestimmen, dass das Testarzneimittel das Kandidatenarzneimittel ist.

## Revendications

1. Composition pharmaceutique destinée à être utilisée dans un traitement anticancéreux, comprenant le sorafénib ou un sel pharmaceutiquement acceptable de celui-ci et le 3-(3,5-dibromo-4-hydroxy-benzylidène)-5-iodo-1,3-dihydro-indol-2-one ou un sel pharmaceutiquement acceptable de celui-ci.

2. Composition pharmaceutique destinée à être utilisée dans un traitement anticancéreux selon la revendication 1, dans laquelle le sorafénib et le 3-(3,5-dibromo-4-hydroxy-benzylidène)-5-iodo-1,3-dihydro-indol-2-one sont administrés séparément, simultanément ou séquentiellement.

3. Composition pharmaceutique destinée à être utilisée dans un traitement anticancéreux selon la revendication 1, dans laquelle le cancer est un carcinome à cellules rénales, un cancer de la prostate, un cancer du sein, un cancer du poumon, un cancer du col de l'utérus, un cancer de la bouche, un gliome, un carcinome à cellules urothéliales ou un mélanome.

4. Composition pharmaceutique destinée à être utilisée dans un traitement anticancéreux selon la revendication 1, comprenant en outre un véhicule pharmaceutiquement acceptable.

5. Composition pharmaceutique destinée à être utilisée dans un traitement anticancéreux selon la revendication 4, dans laquelle les véhicules sont choisis parmi le groupe consistant en excipients, diluants, épaississants, charges, liants, désintégrants, lubrifiants, agents huileux ou non huileux, tensioactifs, agents de suspension, agents gélifiants, adjuvants, conservateurs, antioxydants, stabilisateurs et agents colorants.

6. Composition pharmaceutique destinée à être utilisée dans un traitement anticancéreux selon la revendication 1, dans laquelle la composition pharmaceutique est administrée par voie orale, immersion, injection, applications topiques ou par timbre.

7. Composition pharmaceutique destinée à être utilisée dans un traitement anticancéreux selon la revendication 1, dans laquelle le 3-(3,5-dibromo-4-hydroxy-benzylidène)-5-iodo-1,3-dihydro-indol-2-one modifie la conformation d'une protéine c-Raf présentant la séquence de SEQ ID NO:1 lors de sa liaison à celle-ci, augmentant ainsi l'affinité de liaison du sorafénib par rapport à la protéine c-Raf de manière à dissocier c-Raf du complexe protéique c-Raf/DAPK.

8. Association de composés destinée à être utilisée dans un traitement anticancéreux, dans laquelle l'association comprend le sorafénib et le 3-(3,5-dibromo-4-hydroxy-benzylidène)-5-iodo-1,3-dihydro-indol-2-one, et dans laquelle l'association de composés dissocie un complexe protéique dans les cellules, dans laquelle le complexe protéique est un complexe protéique c-Raf/DAPK, la protéine c-Raf présentant la séquence de SEQ ID NO:1 et la protéine DAPK présentant la séquence de SEQ ID NO:2.

9. Procédé pour cribler un médicament candidat en utilisant un biomarqueur, comprenant les étapes consistant à
fournir un échantillon;
détecter un niveau de phosphorylation du biomarqueur dans l'échantillon avant l'administration d'un médicament expérimental, et détecter une vitesse d'inhibition de la croissance cellulaire du médicament expérimental sur l'échantillon, et lorsque le niveau de phosphorylation du biomarqueur est en corrélation positive avec la vitesse d'inhibition de la croissance cellulaire, déterminer que le médicament expérimental est le médicament candidat, dans lequel,
le biomarqueur est une protéine DAPK présentant la séquence de SEQ ID NO:2, dans lequel le résidu d'acide aminé sérine 308 (Ser308) de la protéine DAPK est phosphorylé.

10. Procédé selon la revendication 9, dans lequel l'échantillon est ascite, sang, urine, fèces, salive, cellules muqueuses, liquide gastrique, bile, ou tissus cancéreux détachés prélevés après une intervention chirurgicale.

11. Procédé selon la revendication 9, dans lequel le médicament candidat comprend le sorafénib et le 3-(3,5-dibromo-4-hydroxy-benzylidène)-5-iodo-1,3-dihydro-indol-2-one.

12. Utilisation d'un biomarqueur lors du criblage d'un médicament candidat, dans laquelle le biomarqueur est une protéine DAPK présentant la séquence de SEQ ID NO:2, dans laquelle le résidu d'acide aminé sérine 308 (Ser308) de la protéine DAPK est phosphorylé.

13. Utilisation selon la revendication 12, dans laquelle le médicament candidat comprend le sorafénib et le 3-(3,5-dibromo-4-hydroxy-benzylidène)-5-iodo-1,3-dihydro-indol-2-one.

14. Utilisation selon la revendication 12, comprenant en outre la détection d'un niveau de phosphorylation du biomarqueur avant l'administration d'un médicament expérimental, et la détection d'une vitesse d'inhibition de la croissance cellulaire du médicament expérimental, et lorsque le niveau de phosphorylation du biomarqueur est en corrélation positive avec la vitesse d'inhibition de la croissance cellulaire, la détermination que le médicament expérimental est le médicament candidat.


### SEQUENCE LISTING

<110> National Defense Medical Center
<120> PHARMACEUTICAL COMPOSITION FOR TREATING CANCER AND BIOMARKER FOR DRUG
   SCREENING
<130> P2900-EP
<150> PCT/CN2014/091712
   <151> 2014-11-19
<160> 6
<170> BiSSAP 1.3
<210> 1
   <211> 648
   <212> PRT
   <213> Homo sapiens
<400> 1
<210> 2
   <211> 1430
   <212> PRT
   <213> Homo sapiens
<400> 2
   Met Thr Val Phe Arg Gln Glu Asn Val Asp Asp Tyr Tyr Asp Thr Gly
   1               5                   10                  15
   Glu Glu Leu Gly Ser Gly Gln Phe Ala Val Val Lys Lys Cys Arg Glu
               20                  25                  30
   Lys Ser Thr Gly Leu Gln Tyr Ala Ala Lys Phe Ile Lys Lys Arg Arg
           35                  40                  45
   Thr Lys Ser Ser Arg Arg Gly Val Ser Arg Glu Asp Ile Glu Arg Glu
       50                  55                  60
   Val Ser Ile Leu Lys Glu Ile Gln His Pro Asn Val Ile Thr Leu His
   65                  70                  75                  80
   Glu Val Tyr Glu Asn Lys Thr Asp Val Ile Leu Ile Leu Glu Leu Val
                   85                  90                  95
   Ala Gly Gly Glu Leu Phe Asp Phe Leu Ala Glu Lys Glu Ser Leu Thr
               100                 105                 110
   Glu Glu Glu Ala Thr Glu Phe Leu Lys Gln Ile Leu Asn Gly Val Tyr
           115                 120                 125
   Tyr Leu His Ser Leu Gln Ile Ala His Phe Asp Leu Lys Pro Glu Asn
       130                 135                 140
   Ile Met Leu Leu Asp Arg Asn Val Pro Lys Pro Arg Ile Lys Ile Ile
   145                 150                 155                 160
   Asp Phe Gly Leu Ala His Lys Ile Asp Phe Gly Asn Glu Phe Lys Asn
                   165                 170                 175
   Ile Phe Gly Thr Pro Glu Phe Val Ala Pro Glu Ile Val Asn Tyr Glu
               180                 185                 190
   Pro Leu Gly Leu Glu Ala Asp Met Trp Ser Ile Gly Val Ile Thr Tyr
           195                 200                 205
   Ile Leu Leu Ser Gly Ala Ser Pro Phe Leu Gly Asp Thr Lys Gln Glu
       210                 215                 220
   Thr Leu Ala Asn Val Ser Ala Val Asn Tyr Glu Phe Glu Asp Glu Tyr
   225                 230                 235                 240
   Phe Ser Asn Thr Ser Ala Leu Ala Lys Asp Phe Ile Arg Arg Leu Leu
                   245                 250                 255
   Val Lys Asp Pro Lys Lys Arg Met Thr Ile Gln Asp Ser Leu Gln His
               260                 265                 270
   Pro Trp Ile Lys Pro Lys Asp Thr Gln Gln Ala Leu Ser Arg Lys Ala
           275                 280                 285
   Ser Ala Val Asn Met Glu Lys Phe Lys Lys Phe Ala Ala Arg Lys Lys
       290                 295                 300
   Trp Lys Gln Ser Val Arg Leu Ile Ser Leu Cys Gln Arg Leu Ser Arg
   305                 310                 315                 320
   Ser Phe Leu Ser Arg Ser Asn Met Ser Val Ala Arg Ser Asp Asp Thr
                   325                 330                 335
   Leu Asp Glu Glu Asp Ser Phe Val Met Lys Ala Ile Ile His Ala Ile
               340                 345                 350
   Asn Asp Asp Asn Val Pro Gly Leu Gln His Leu Leu Gly Ser Leu Ser
           355                 360                 365
   Asn Tyr Asp Val Asn Gln Pro Asn Lys His Gly Thr Pro Pro Leu Leu
       370                 375                 380
   Ile Ala Ala Gly Cys Gly Asn Ile Gln Ile Leu Gln Leu Leu Ile Lys
   385                 390                 395                 400
   Arg Gly Ser Arg Ile Asp Val Gln Asp Lys Gly Gly Ser Asn Ala Val
                   405                 410                 415
   Tyr Trp Ala Ala Arg His Gly His Val Asp Thr Leu Lys Phe Leu Ser
               420                 425                 430
   Glu Asn Lys Cys Pro Leu Asp Val Lys Asp Lys Ser Gly Glu Met Ala
           435                 440                 445
   Leu His Val Ala Ala Arg Tyr Gly His Ala Asp Val Ala Gln Leu Leu
       450                 455                 460
   Cys Ser Phe Gly Ser Asn Pro Asn Ile Gln Asp Lys Glu Glu Glu Thr
   465                 470                 475                 480
   Pro Leu His Cys Ala Ala Trp His Gly Tyr Tyr Ser Val Ala Lys Ala
                   485                 490                 495
   Leu Cys Glu Ala Gly Cys Asn Val Asn Ile Lys Asn Arg Glu Gly Glu
               500                 505                 510
   Thr Pro Leu Leu Thr Ala Ser Ala Arg Gly Tyr His Asp Ile Val Glu
           515                 520                 525
   Cys Leu Ala Glu His Gly Ala Asp Leu Asn Ala Cys Asp Lys Asp Gly
       530                 535                 540
   His Ile Ala Leu His Leu Ala Val Arg Arg Cys Gln Met Glu Val Ile
   545                 550                 555                 560
   Lys Thr Leu Leu Ser Gln Gly Cys Phe Val Asp Tyr Gln Asp Arg His
                   565                 570                 575
   Gly Asn Thr Pro Leu His Val Ala Cys Lys Asp Gly Asn Met Pro Ile
               580                 585                 590
   Val Val Ala Leu Cys Glu Ala Asn Cys Asn Leu Asp Ile Ser Asn Lys
           595                 600                 605
   Tyr Gly Arg Thr Pro Leu His Leu Ala Ala Asn Asn Gly Ile Leu Asp
       610                 615                 620
   Val Val Arg Tyr Leu Cys Leu Met Gly Ala Ser Val Glu Ala Leu Thr
   625                 630                 635                 640
   Thr Asp Gly Lys Thr Ala Glu Asp Leu Ala Arg Ser Glu Gln His Glu
                   645                 650                 655
   His Val Ala Gly Leu Leu Ala Arg Leu Arg Lys Asp Thr His Arg Gly
               660                 665                 670
   Leu Phe Ile Gln Gln Leu Arg Pro Thr Gln Asn Leu Gln Pro Arg Ile
           675                 680                 685
   Lys Leu Lys Leu Phe Gly His Ser Gly Ser Gly Lys Thr Thr Leu Val
       690                 695                 700
   Glu Ser Leu Lys Cys Gly Leu Leu Arg Ser Phe Phe Arg Arg Arg Arg
   705                 710                 715                 720
   Pro Arg Leu Ser Ser Thr Asn Ser Ser Arg Phe Pro Pro Ser Pro Leu
                   725                 730                 735
   Ala Ser Lys Pro Thr Val Ser Val Ser Ile Asn Asn Leu Tyr Pro Gly
               740                 745                 750
   Cys Glu Asn Val Ser Val Arg Ser Arg Ser Met Met Phe Glu Pro Gly
           755                 760                 765
   Leu Thr Lys Gly Met Leu Glu Val Phe Val Ala Pro Thr His His Pro
       770                 775                 780
   His Cys Ser Ala Asp Asp Gln Ser Thr Lys Ala Ile Asp Ile Gln Asn
   785                 790                 795                 800
   Ala Tyr Leu Asn Gly Val Gly Asp Phe Ser Val Trp Glu Phe Ser Gly
                   805                 810                 815
   Asn Pro Val Tyr Phe Cys Cys Tyr Asp Tyr Phe Ala Ala Asn Asp Pro
               820                 825                 830
   Thr Ser Ile His Val Val Val Phe Ser Leu Glu Glu Pro Tyr Glu Ile
           835                 840                 845
   Gln Leu Asn Gln Val Ile Phe Trp Leu Ser Phe Leu Lys Ser Leu Val
       850                 855                 860
   Pro Val Glu Glu Pro Ile Ala Phe Gly Gly Lys Leu Lys Asn Pro Leu
   865                 870                 875                 880
   Gln Val Val Leu Val Ala Thr His Ala Asp Ile Met Asn Val Pro Arg
                   885                 890                 895
   Pro Ala Gly Gly Glu Phe Gly Tyr Asp Lys Asp Thr Ser Leu Leu Lys
               900                 905                 910
   Glu Ile Arg Asn Arg Phe Gly Asn Asp Leu His Ile Ser Asn Lys Leu
           915                 920                 925
   Phe Val Leu Asp Ala Gly Ala Ser Gly Ser Lys Asp Met Lys Val Leu
       930                 935                 940
   Arg Asn His Leu Gln Glu Ile Arg Ser Gln Ile Val Ser Val Cys Pro
   945                 950                 955                 960
   Pro Met Thr His Leu Cys Glu Lys Ile Ile Ser Thr Leu Pro Ser Trp
                   965                 970                 975
   Arg Lys Leu Asn Gly Pro Asn Gln Leu Met Ser Leu Gln Gln Phe Val
               980                 985                 990
   Tyr Asp Val Gln Asp Gln Leu Asn Pro Leu Ala Ser Glu Glu Asp Leu
           995                 1000                1005
   Arg Arg Ile Ala Gln Gln Leu His Ser Thr Gly Glu Ile Asn Ile Met
       1010                1015                1020
   Gln Ser Glu Thr Val Gln Asp Val Leu Leu Leu Asp Pro Arg Trp Leu
   1025                1030                1035                1040
   Cys Thr Asn Val Leu Gly Lys Leu Leu Ser Val Glu Thr Pro Arg Ala
                   1045                1050                1055
   Leu His His Tyr Arg Gly Arg Tyr Thr Val Glu Asp Ile Gln Arg Leu
               1060                1065                1070
   Val Pro Asp Ser Asp Val Glu Glu Leu Leu Gln Ile Leu Asp Ala Met
           1075                1080                1085
   Asp Ile Cys Ala Arg Asp Leu Ser Ser Gly Thr Met Val Asp Val Pro
       1090                1095                1100
   Ala Leu Ile Lys Thr Asp Asn Leu His Arg Ser Trp Ala Asp Glu Glu
   1105                1110                1115                1120
   Asp Glu Val Met Val Tyr Gly Gly Val Arg Ile Val Pro Val Glu His
                   1125                1130                1135
   Leu Thr Pro Phe Pro Cys Gly Ile Phe His Lys Val Gln Val Asn Leu
               1140                1145                1150
   Cys Arg Trp Ile His Gln Gln Ser Thr Glu Gly Asp Ala Asp Ile Arg
           1155                1160                1165
   Leu Trp Val Asn Gly Cys Lys Leu Ala Asn Arg Gly Ala Glu Leu Leu
       1170                1175                1180
   Val Leu Leu Val Asn His Gly Gln Gly Ile Glu Val Gln Val Arg Gly
   1185                1190                1195                1200
   Leu Glu Thr Glu Lys Ile Lys Cys Cys Leu Leu Leu Asp Ser Val Cys
                   1205                1210                1215
   Ser Thr Ile Glu Asn Val Met Ala Thr Thr Leu Pro Gly Leu Leu Thr
               1220                1225                1230
   Val Lys His Tyr Leu Ser Pro Gln Gln Leu Arg Glu His His Glu Pro
           1235                1240                1245
   Val Met Ile Tyr Gln Pro Arg Asp Phe Phe Arg Ala Gln Thr Leu Lys
       1250                1255                1260
   Glu Thr Ser Leu Thr Asn Thr Met Gly Gly Tyr Lys Glu Ser Phe Ser
   1265                1270                1275                1280
   Ser Ile Met Cys Phe Gly Cys His Asp Val Tyr Ser Gln Ala Ser Leu
                   1285                1290                1295
   Gly Met Asp Ile His Ala Ser Asp Leu Asn Leu Leu Thr Arg Arg Lys
               1300                1305                1310
   Leu Ser Arg Leu Leu Asp Pro Pro Asp Pro Leu Gly Lys Asp Trp Cys
           1315                1320                1325
   Leu Leu Ala Met Asn Leu Gly Leu Pro Asp Leu Val Ala Lys Tyr Asn
       1330                1335                1340
   Thr Ser Asn Gly Ala Pro Lys Asp Phe Leu Pro Ser Pro Leu His Ala
   1345                1350                1355                1360
   Leu Leu Arg Glu Trp Thr Thr Tyr Pro Glu Ser Thr Val Gly Thr Leu
                   1365                1370                1375
   Met Ser Lys Leu Arg Glu Leu Gly Arg Arg Asp Ala Ala Asp Phe Leu
               1380                1385                1390
   Leu Lys Ala Ser Ser Val Phe Lys Ile Asn Leu Asp Gly Asn Gly Gln
           1395                1400                1405
   Glu Ala Tyr Ala Ser Ser Cys Asn Ser Gly Thr Ser Tyr Asn Ser Ile
       1410                1415                1420
   Ser Ser Val Val Ser Arg
   1425                1430
<210> 3
   <211> 21
   <212> RNA
   <213> Artificial Sequence
<220>
   <223> synthetic
<400> 3
   caagaaacgu uagcaaaugu u 21
<210> 4
   <211> 21
   <212> RNA
   <213> Artificial Sequence
<220>
   <223> synthetic
<400> 4
   cauuugcuaa cguuucuugu u 21
<210> 5
   <211> 21
   <212> RNA
   <213> Artificial Sequence
<220>
   <223> synthetic
<400> 5
   ggucaaggau ccaaagaagu u 21
<210> 6
   <211> 21
   <212> RNA
   <213> Artificial Sequence
<220>
   <223> synthetic
<400> 6
   cuucuuugga uccuugaccu u 21